# EUROPEAN PATENT APPLICATION

(11) **EP 4 821 521 A1**
(43) Date of publication of application: **14.10.2026**
(21) Application number: 23960522.3
(22) Date of filing: 07.12.2023
(51) Int. Cl.: A61M 5/20, A61M 5/31, A61M 5/315, A61M 5/32

(54) **AUTO-INJECTOR**

(71) Applicant: Zhejiang Summed Medtech Co., Ltd., Huzhou, Zhejiang 313000 (CN)
(72) Inventor: GAO, Pengtao, Jiaxing, Zhejiang 314100 (CN); WENG, Chenjun, Jiaxing, Zhejiang 314100 (CN)
(74) Representative: Schwerbrock, Florian
(86) International application number: PCT/CN2023/136949
(87) International publication number: WO 2025/118210

(57) **Abstract**

The present invention discloses an auto-injector, comprising: a cap and a front housing, and a compression mechanism is disposed inside the cap and the front housing. The compression mechanism includes a rear housing. A latching block on a surface of the plunger rotates synchronously with the plunger, thereby disengaging the latching block from a guide track. The plunger spring inside the plunger is normally maintained in a pre-compressed state, is disposed within the plunger. Once the latching block is disengaged, the plunger spring drives the plunger forward to push the rubber stopper. This, in turn, squeezes the liquid medication within the syringe, causing the liquid medication to flow through the needle and inject into the patient's body. By utilizing streamlined components, the invention reduces manufacturing costs, enhances structural stability of the injector, and fulfills the market demand for a compact form factor.

## Description

### FIELD OF THE INVENTION

The present invention relates to a medical device technical field, particularly relates to an auto-injector.

### BACKGROUND OF THE INVENTION

The auto-injector is a product of modern medical technology that can greatly change people's perception of injection therapy. The original intention of this medical device is to simply the injection procedure, reduce the difficulty of operation, reduce the pain of patients, and better control the dosage and the injection speed of drug.

Due to their automated features, the auto-injectors are widely favored by medical professionals and patients alike. The manufacturing process is highly sophisticated, demanding extreme precision and quality for every component. This is because the precision of the auto-injector directly determines its performance and clinical efficacy. Consequently, each component must undergo rigorous testing and inspection to ensure it adheres to design specifications and quality standards.

To meet above requirements, the manufacturing of auto- injector typically requires high-quality materials. These materials must not only be durable enough to maintain their shape and precision through repeated use but also reliable enough to ensure accurate dosing. These factors put pressure on manufacturing costs, potentially increasing the cost of raw materials.

Therefore, to ensure the product quality and performance, precision components must be crafted from high-quality materials to guarantee durability and accuracy, which consequently increases raw materials expenses.

### SUMMARY OF THE INVENTION

An object of the present invention is to provide an auto-injector which is provided for solving the problem raised in the background technology. In order to achieve the above object, the present invention provides the following technical. The auto-injector comprises:
a cap and a front housing;
a compression mechanism, which is disposed inside the cap and the front housing;
the compression mechanism comprises a rear housing fixedly connected to one end of the front housing, a rear cap is disposed inside the rear housing, a braking sleeve movably connected inside the rear cap, the braking spring is movably sleeve on a surface of the braking sleeve, and a trigger sleeve located inside the front housing is fixedly installed on one end of the braking sleeve;
a latching mechanism, which is disposed inside the braking sleeve, wherein the latching mechanism comprises a fixed sleeve rotatably connected inside the braking sleeve, a guide track is defined on a surface of the fixed sleeve, a syringe sleeve is fixedly installed on one end of the fixed sleeve, and a resilient arm is movably connected within the guide track;
a trigger mechanism, which is disposed inside the fixed sleeve, wherein the trigger mechanism comprises a plunger movably disposed within the fixed sleeve, a latching block is located inside the trach is fixedly connected to a surface of the plunger, and a plunger spring is movably connected inside the plunger spring; and
an injection assembly, which is disposed inside the syringe sleeve, and the injection assembly comprises a syringe;
wherein the latching block is configured to rotate under the pushing of the trigger sleeve, such that the latching block is driven to move from a left end to a right end of the resilient arm, and a rubber stopper squeezes the liquid medication within the syringe under the elastic force of the plunger spring.

Preferably, the syringe is fixedly connected to inside the syringe sleeve, and the rubber stopper inside the syringe is fixedly installed on one end of the plunger.

Preferably, one end of the braking spring is fixedly connected to one end of the rear cap, and the other end of the braking spring is fixedly connected to the braking sleeve; braking spring is configured to be compressed on a surface of the braking sleeve.

Preferably, the resilient arm being of an inclined configuration.

Preferably, one end of the plunger spring is fixedly connected to inner end of the fixed sleeve, the other end of the plunger spring is fixedly connected to one end of the plunger, and the plunger spring is normally in a compressed state.

Preferably, the fixed sleeve further comprises an anti-retraction clip, the braking sleeve is driven to move by pushing of the trigger sleeve, thereby compressing the braking spring and urging the latching block to move rightward, subsequently, the latching block is biased by the accumulated force of the liquid medication, wherein the anti-retraction clip crosses a shoulder to achieve the anti-back function, such that the trigger sleeve is prevented from moving backward.

Preferably, the trigger sleeve is disposed inside the front housing and is configured to move within the front housing.

Preferably, a PFS tip cap is installed inside the cap, and a clamping block is disposed therein; one end of the PFS tip cap abuts against the clamping block.

Preferably, a diameter of the syringe sleeve is larger than that of the syringe sleeve, and the syringe sleeve is disposed inside the trigger sleeve.

The beneficial effects of the present invention are as follows:
The present invention facilitates the injection process through the movement of the trigger sleeve, which cause the needle at one end of the syringe to abut against and penetrate the patient's skin. During this movement, the trigger sleeve pushes the braking sleeve, thereby compressing the braking spring. The action causes the plunger to rotate, allowing the latching blocks on its surface to rotate concurrently until they are disengaged from a guide track.

The plunger spring, which is normally maintained in a compressed state, is disposed within the plunger. Once the latching block is disengaged, the plunger spring drives the plunger forward to push the rubber stopper. This, in turn, squeezes the liquid medication within the syringe, causing it to flow through the needle and into the patient's body.

By utilizing streamlined components, the invention reduces manufacturing costs, enhances structural stability of the injector, and fulfills the market demand for a compact form factor.

### BRIEF DESCRIPTION OF THE DRAWINGS

Fig.1 is a schematic diagram of a syringe in accordance with the present invention.
Fig.2 is a cross-sectional view of the cap in accordance with the present invention.
Fig.3 is a cross-sectional view of the trigger sleeve in accordance with present invention.
Fig.4 is a cross-sectional view of braking sleeve in accordance with the present invention.
Fig.5 is a cross-sectional view of the syringe in accordance with the present invention.
Fig.6 is a schematic diagram of the trigger sleeve after being triggered in accordance with the present invention.

In the figure:
1 cap; 2 front housing; 3 trigger sleeve; 4 syringe sleeve; 5 fixed sleeve; 6 braking sleeve; 7 rear housing; 8 rear cap; 9 braking spring; 10 plunger spring; 11 plunger; 12 rubber stopper; 13 syringe; 14 PFS tip cap; 15 guide track; 16 latching block; 17 resilient arm; 18 clamping block; 19 anti-retraction clip.

### DETAILED DESCRIPTION OF THE PREFERRED EMBODIMENT

Since the various aspects and embodiments are merely illustrative and not limiting, those skilled in the art may, after reading this specification, conceive other aspects and embodiments without departing from the scope of the present invention. The following detailed description and claims will further highlight the features and advantages of these embodiments.

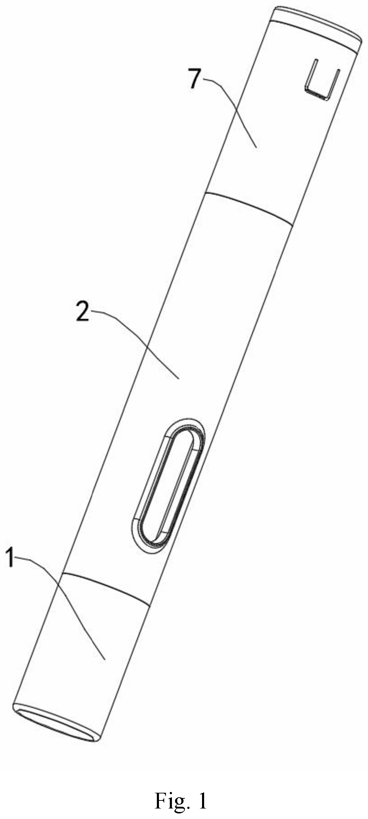

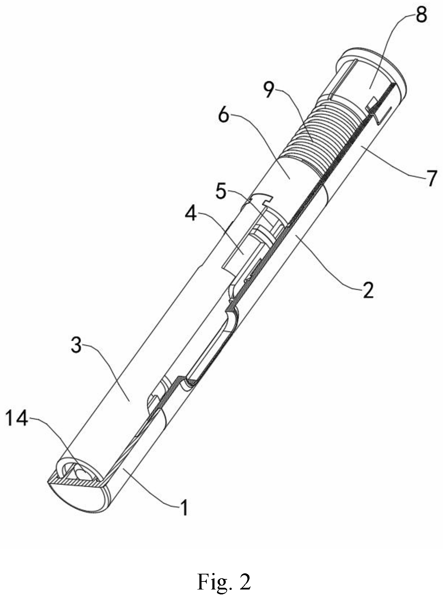

As shown in Fig. 1 to Fig. 6, the present invention provides an auto-injector, comprises: a cap 1 and a front housing 2, a PFS tip cap is installed inside the cap 1. A compression mechanism disposed inside the cap 1 and the front housing 2, and further including a rear housing 7 fixedly connected to one end of the front housing 2. A rear cap 8 is disposed inside the rear housing 7, and a braking sleeve 6 is movably connected inside the rear cap 8. A braking sleeve 9 is movably sleeved on a surface of the braking sleeve6, and a trigger sleeve 3 located inside the front housing 2 is fixedly connected to one end of the braking sleeve 6.

A latching mechanism disposed inside the braking sleeve 6 and including a fixed sleeve 5 rotatably connected inside the braking sleeve 6. A guide track 15 is defined on a surface of the fixed sleeve 5, and a syringe sleeve 4 is fixedly installed on one end of the fixed sleeve 5.

A trigger mechanism disposed inside the fixed sleeve 5 and including a plunger 11 movably connected inside fixed sleeve 5. A latching block 16 is located inside the guide track 15 is fixedly connected to a surface of the plunger 11, and a plunger spring 10 is movably connected inside the plunger 11.

An injection assembly disposed inside the syringe sleeve 4 and including a syringe 13.

The latching block 16 is configured to rotate under the pushing the trigger sleeve 3, such that the latching block 16 is driven to move from a left end to a right end of a resilient arm 17, and a rubber stopper 12 squeezes the liquid medication within the syringe 13 under the elastic force of the plunger spring 10.

As the operator pulls off the cap 1, and the PFS tip cap 14 is removed simultaneously. A surface of the trigger sleeve 3 abuts against the patient's skin. Then, the trigger sleeve 3 is driven to move to allow the needle of the injection assembly penetrates the patient's skin during this movement. In addition, during this movement, the trigger sleeve 3 pushes the braking sleeve 6, thereby compressing the braking spring 9. This action causes the plunger 11 to rotate, allowing the latching block 16 moves inside the guide track 15 until the disengagement of the latching block 16 from the guide track 15. Thereafter, the compressed plunger spring 10 pushes the plunger 11 to inject the liquid medication within the injection assembly into the patient's body.

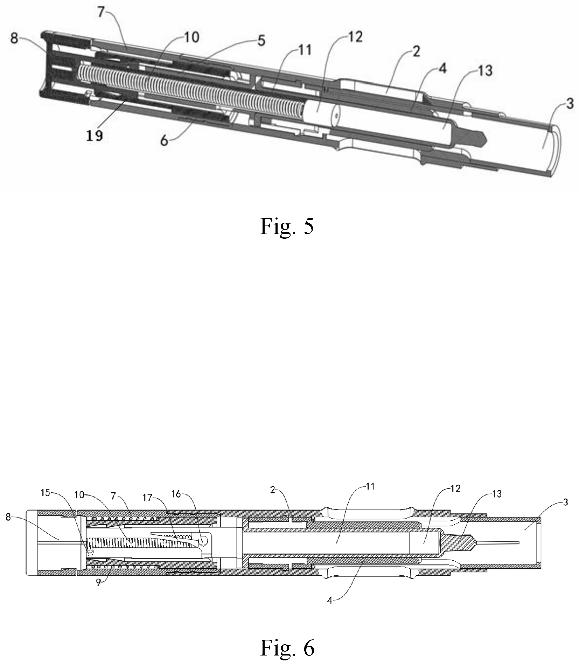

As shown in Fig. 5, the syringe 13 is fixedly connected inside the syringe sleeve 4 and the rubber stopper 12 inside the syringe 13 is fixedly installed on one end of the plunger 11.

Through the design of the injection assembly, when the limit of the latching block 15 is disengaged, the plunger spring 10 pushes the plunger 11 and the latching block 16 to move, and the plunger 11 is driven to push inside the syringe 13, such that the rubber stopper 12 pushes the liquid medication to flow and inject into the patient's body.

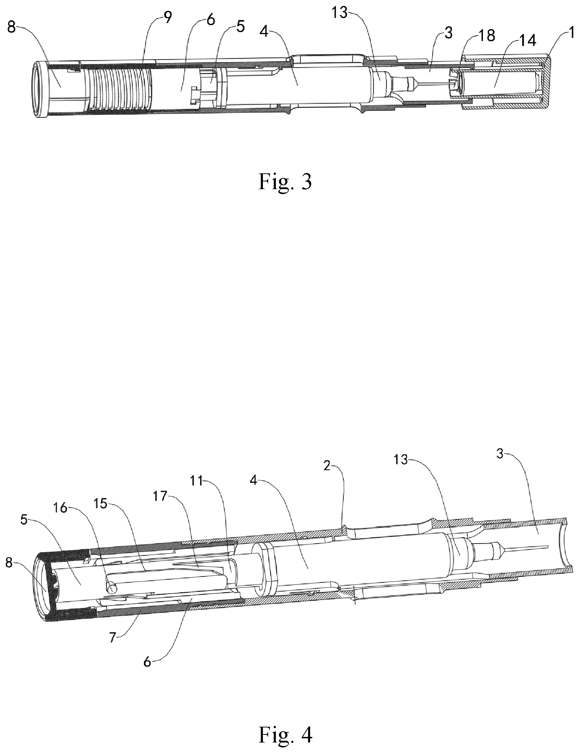

As shown in Fig. 3, one end of the braking spring 9 is fixedly connected to one end of the rear cap 8 and the other end of the braking spring 9 is fixedly connected to the braking sleeve, such that the braking spring 9 is configured to be compressed on a surface of the braking sleeve 6.

Through the design of the braking spring 9, during the movement of the braking spring 9, the rear cap 8 is fixed inside the rear housing 7 to serve as a support point, and a groove disposed on the surface of the braking sleeve 6 to fixedly connect the other end of the braking spring 9, such that the braking sleeve 6 is driven to squeeze the braking spring 9 to compress the braking spring 9 smoothly.

As shown in Fig. 5, a guide groove is provided on inner wall of the braking sleeve 6 and an anti-retraction clip 19 is disposed inside the fixed sleeve 5.

The braking sleeve 6 is driven to move by pushing the trigger sleeve 3, thereby compressing the braking spring 9 and urging the latching block to move rightward, subsequently, the latching block 16 is biased by the accumulated force of the plunger spring 10 to move forward to complete the injection of liquid medication, and the anti-retraction clip 19 crosses a shoulder to achieve the anti-back function, such that the trigger sleeve 3 is prevented from moving backward for protecting the needle.

As shown in Fig. 4, through the design of the resilient arm 17, which is located inside the guide track 15, the latching block 16 abuts against the surface of the resilient arm 17 to push the movement of the resilient arm 17, allowing the latching block 16 pass through the surface of the resilient arm 17 during the movement of the latching block 16. Once the latching block 16 moves away from the surface of the resilient arm 17, the resilient arm 17 will return to its original position, thereby preventing the latching block 16 from backward.

As shown in Fig. 5, one end of the plunger spring 10 is fixedly connected inside the fixed sleeve 5, the other end of the plunger spring 10 is fixedly connected to inside the rear cap 8, and the plunger spring 10 is normally in a compressed state.

Through the design of the plunger spring 10, the plunger spring 10 is normally in a compressed state when the pushing spring 10 is installed. The guide track 15 disengages the compression of the latching block 16, the compressed plunger spring 10 is moved inside the fixed sleeve 5, and the plunge r11 is driven to move the rubber stopper 12 to inject the liquid medication into the patient's skin.

As shown in Fig. 5, a surface of the rubber stopper 12 matches inside the syringe 13, such that the rubber stopper 12 can move inside the syringe 13.

Through the design of the rubber stopper 12, when the rubber stopper 12 moves, the rubber stopper 12 is matched the inner wall of the syringe 13, and can move inside the syringe 13, such that the rubber stopper 12 will squeeze the liquid medication within the syringe 13 and flow through the needle and inject into the patient's body.

As shown in Fig. 4, the trigger sleeve 3 is located inside the front housing 2 and is moved inside the front housing 2.

Through the design of the trigger sleeve 3, the trigger sleeve 3 is provided to encapsulate the needle at one end of the syringe 13 before activation of the trigger sleeve 3. Upon the movement of the trigger sleeve 3, the needle protrudes to facilitate the injection into the patient's skin.

As shown in Fig. 3, a clamping block 18 is disposed inside the cap 1 and one end of a PFS tip cap 14 abuts against the clamping block 18.

Through the design of the PFS tip cap 14, when the cap 1 moves, the PFS tip cap 14 is designed to be interlocked with the clamping block 18, thereby allowing the PFS tip cap 14 is removed concurrently with the movement of the cap 1.

As shown in Fig. 3, the diameter of the syringe sleeve 4 is larger than that of the syringe 13, and the syringe sleeve 4 is located inside the trigger sleeve 3.

Through the design of the syringe sleeve 4, the syringe sleeve 4 is configured to support and secure the syringe 13, thereby preventing internal displacement of the syringe 13 during the injection of liquid medication.

Working principle and Operating Procedure:
To use the syringe, the user first removes the cap 1, which simultaneously detaches the PFS tip cap 14 from the end of the syringe 13. When one end of the trigger sleeve 3 is pressed against the patient's skin, the trigger sleeve 3 is retracted, allowing the needle at one end of the syringe to abut against and penetrate the patient's skin.

The movement of the trigger sleeve 3 drives braking sleeve 6, which in turn compresses the braking spring 9. This action rotates the plunger 11, carrying the latching block 16 on the surface of the plunger 11 with it. Consequently, the guide track 15 disengages from the latching block 16. Since the plunger spring 10 is held in a pre-compressed state inside the plunger 11, the plunger 11 is driven to move by compressing the plunger spring 10 once the latching block 16 is disengaged.

Then, the plunger 11 drives to move the rubber stopper 12 forward to compress the liquid medication within the syringe 13, causing the liquid medication to flow through the needle and inject into the patient's body. Once the injection is complete and the needle is withdrawn from the patient's skin, the trigger sleeve 3 is disengaged from compression. The compressed braking spring 9 pushes the braking sleeve 6 and the trigger sleeve 3 forward, ensuring the trigger sleeve 3 encapsulates the needle for safety.

In the disclosure of the present invention, terms such as "first" or "second," and similar ordinal numbers are primarily used to distinguish or reference identical or similar components or structures and do not necessarily imply any spatial or temporal order of these components or structures. In certain cases, or configurations, the ordinal terms can be used interchangeably without affecting the implementation of the present invention.

In the disclosure of the present invention, terms such as "comprising," "having," or any other similar expressions are intended to encompass non-exclusive inclusivity. For example, a component or structure containing multiple elements is not limited to the elements explicitly listed herein but may also include other elements that are not explicitly mentioned but are inherently typical of such a component or structure.

While the invention has been described in terms of what is presently considered to be the most practical and preferred embodiments, it is to be understood that the invention need not be limited to the disclosed embodiments. On the contrary, it is intended to cover various modifications and similar arrangements included within the spirit and scope of the appended claims which are to be accorded with the broadest interpretation so as to encompass all such modifications and similar structures.

## Claims

1. An auto-injector, the **characterized in that** the auto-injector comprising:
a cap (1) and a front housing (2);
a compression mechanism, which is disposed inside the cap (1) and the front housing (2), wherein the compression mechanism comprises a rear housing (7) fixedly connected to one end of the front housing (2), a rear cap (8) is disposed inside the rear housing (7), a braking sleeve (6) movably connected inside the rear cap (8), a braking spring (9) is movably sleeved on a surface of the braking sleeve (6), and a trigger sleeve (3) located inside the front housing (2) is fixedly installed on one end of the braking sleeve (6);
a latching mechanism, which is disposed inside the braking sleeve (6), wherein the latching mechanism comprises a fixed sleeve (5) rotatably connected inside the braking sleeve (6), a guide track (15) is defined on a surface of the fixed sleeve (5), a syringe sleeve (4) is fixedly installed on one end of the fixed sleeve (5), and a resilient arm (17) is movably connected within the guide track (15);
a trigger mechanism, which is disposed inside the fixed sleeve (5), wherein the trigger mechanism comprises a plunger (11) movably disposed within the fixed sleeve (5), a latching block (16) is located inside the guide track (15) is fixedly connected to a surface of the plunger (11), and a plunger spring (10) is movably connected inside the plunger (11); and
an injection assembly, which is disposed inside the syringe sleeve (4), and the injection assembly comprises a syringe (13);
wherein the latching block (16) is configured to rotate under the pushing of the trigger sleeve (3), such that the latching block (16) is driven to move from a left end to a right end of the resilient arm (17), and a rubber stopper (12) squeezes the liquid medication within the syringe (13) under the elastic force of the plunger spring (10);
wherein one end of the braking spring (9) is fixedly connected to one end of the rear cap (8), and the other end of the braking spring (9) is fixedly connected to the braking sleeve (6); the braking spring (9) is configured to be compressed on a surface of the braking sleeve (6), and when the trigger sleeve (3) is disengaged from compression, the braking spring (9) pushes the braking sleeve (6) and the trigger sleeve (3) forward, ensuring the trigger sleeve (3) encapsulates the needle.

2. The auto-injector according to claim 1, the **characterized in that**: the syringe (13) is fixedly connected to inside the syringe sleeve (4), and the rubber stopper (12) inside the syringe (13) is fixedly installed on one end of the plunger (11).

3. The auto-injector according to claim 1, the **characterized in that**: the resilient arm (17) being of an inclined configuration.

4. The auto-injector according to claim 1, the **characterized in that**: one end of the plunger spring (10) is fixedly connected to inner end of the fixed sleeve (5), the other end of the plunger spring (10) is fixedly connected to one end of the plunger (11), and the plunger spring (10) is normally in a pre-compressed state.

5. The auto-injector according to claim 1, the **characterized in that**: the fixed sleeve (5) further comprises an anti-retraction clip (19), the braking sleeve (6) is driven to move by pushing of the trigger sleeve (3), thereby compressing the braking spring (9) and urging the latching block (16) to move rightward, subsequently, the latching block (16) is biased by the accumulated force of the plunger spring (10) to move forward to complete the injection of the liquid medication, wherein the anti-retraction clip (19) crosses a shoulder to achieve the anti-back function, such that the trigger sleeve (3) is prevented from moving backward.

6. The auto-injector according to claim 1, the **characterized in that**: the trigger sleeve (3) is disposed inside the front housing (2) and is configured to move within the front housing (2).

7. The auto-injector according to claim 1, the **characterized in that**: a PFS tip cap (14) is installed inside the cap (1), and a clamping block (18) is disposed therein; one end of the PFS tip cap (14) abuts against the clamping block (18).

8. The auto-injector according to claim 1, the **characterized in that**: a diameter of the syringe sleeve (4) is larger than that of the syringe sleeve (13), and the syringe sleeve (4) is disposed inside the trigger sleeve (3).
